(19) Europäisches Patentamt
European Patent Office
Office européen des brevets

(11) Veröffentlichungsnummer : **0 094 487**
**B1**

(12) # EUROPÄISCHE PATENTSCHRIFT

(45) Veröffentlichungstag der Patentschrift : **09.09.87**

(21) Anmeldenummer : **83102646.3**

(22) Anmeldetag : **17.03.83**

(51) Int. Cl.[4] : **C 09 K 19/30**, C 07 C 13/28, C 07 C 39/17, C 07 C 43/21, C 07 C 69/017, C 07 C 69/76, C 07 C121/64, C 07 C121/75, G 02 F 1/13

(54) **Hydroterphenyle, ihre Herstellung und Verwendung für flüssigkristalline Dielektrika.**

(30) Priorität : **30.03.82 DE 3211601**

(43) Veröffentlichungstag der Anmeldung : **23.11.83 Patentblatt 83/47**

(45) Bekanntmachung des Hinweises auf die Patenterteilung : **09.09.87 Patentblatt 87/37**

(84) Benannte Vertragsstaaten : **AT CH DE FR GB IT LI NL**

(56) Entgegenhaltungen :
**EP-A- 0 019 665**
**EP-A- 0 022 183**
**EP-A- 0 030 277**
**EP-A- 0 062 470**
**DE-A- 2 701 591**
**DE-A- 3 139 130**
**Die Akte enthält technische Angaben, die nach dem Eingang der Anmeldung eingereicht wurden und die nicht in dieser Patentschrift enthalten sind.**

(73) Patentinhaber : **Merck Patent Gesellschaft mit beschränkter Haftung**
**Frankfurter Strasse 250**
**D-6100 Darmstadt (DE)**

(72) Erfinder : **Römer, Michael, Dr.**
**Im Grossen Garten 18**
**D-6054 Rodgau 2 (DE)**
Erfinder : **Krause, Joachim, Dr.**
**Samuel-Morse-Strasse 14**
**D-6110 Dieburg (DE)**
Erfinder : **Weber, Georg**
**Wilhelm-Leuschner-Strasse 38**
**D-6106 Erzhausen (DE)**
Erfinder : **Eidenschink, Rudolf, Dr.**
**Erlenweg 17**
**D-6110 Dieburg (DE)**

**EP 0 094 487 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue Hydroterphenyle der Formel I

$$R^1—Cy—Cy—Ph—R^2 \qquad (I)$$

worin
$R^1$ Alkyl,
$R^2$ H, OH, CN, Alkyl, —O-Alkyl, —$COOR^3$ oder —O—CO—$R^3$,
$R^3$ H, Alkyl, —Cy-Alkyl, —Ph-Alkyl, —Ph—O-Alkyl, —Ph—CN oder —Ph—F,
Cy 1,4-Cyclohexylen und
Ph 1,4-Phenylen
bedeuten, wobei die Alkylgruppen jeweils 1-10 C-Atome enthalten.

Diese Substanzen können wie ähnliche, z. B. aus der DE-OS 29 33 563 bekannte Verbindungen als Komponenten flüssigkristalliner Dielektrika verwendet werden, insbesondere für Displays, die auf dem Prinzip der verdrillten Zelle beruhen.

EP-OS 0 019 665 betrifft Flüssigkristallverbindungen, die eine Variation der $\varepsilon_\perp$-Werte oberhalb des Wertes 5 und damit eine wesentlich verbesserte Anpassung an die Betriebserfordernisse von FK-Zellen durch freiere Variation der $\Delta\varepsilon$-Werte gestatten, wobei dieses Problem gelöst wird durch Einführung lateraler Substituenten ($X^2$ bis $X^4$) in die beschriebenen Verbindungen. Die hierdurch bedingte Querpolarisierung senkrecht zur Molekülachse ermöglicht $\varepsilon_\perp$-Werte oberhalb des Wertes 5.

In der EP-OS 0 019 665 ist eine sehr breite allgemeine Formel angegeben, die einige der Verbindungen der Formel I (nämlich diejenigen mit $R^2$ = Halogen) umschließt. Jedoch ist an keiner Stelle dieser EP-OS ein Hinweis zu finden, durch den die Herstellung und Verwendung dieser speziellen Verbindungen ohne laterale Substitution nahegelegt wird. Die Verbindungen der Formel I weisen dementsprechend auch keine $\varepsilon_\perp$-Werte oberhalb des Wertes 5 auf und betreffen ein vollständig verschiedenes Teilgebiet flüssigkristalliner Verbindungen.

Der Erfindung lag die Aufgabe zugrunde, neue stabile flüssigkristalline Verbindungen aufzufinden, die als Komponenten flüssigkristalliner Dielektrika geeignet sind, insbesondere für nematische Phasen mit breitem nematischem Bereich und niedriger Viskosität. Diese Aufgabe wurde mit der Bereitstellung der Verbindungen der Formel I gelöst.

Es wurde gefunden, daß die Verbindungen der Formel I als Komponenten flüssigkristalliner Dielektrika vorzüglich geeignet sind. Insbesondere sind mit ihrer Hilfe stabile flüssigkristalline Phasen mit breitem nematischem Bereich und niedriger Viskosität herstellbar.

Mit der Bereitstellung der Verbindungen der Formel I wird außerdem ganz allgemein die Palette der flüssigkristallinen Substanzen, die sich unter verschiedenen anwendungstechnischen Gesichtspunkten zur Herstellung nematischer Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Dielektrika zum überwiegenden Teil zusammengesetzt sind; es können aber auch Verbindungen der Formel I flüssigkristallinen Basismaterialien aus anderen Verbindungsklassen zugesetzt werden, um beispielsweise die Viskosität eines solchen Dielektrikums zu senken. Die Verbindungen der Formel I, insbesondere diejenigen, worin $R^2$ H, OH, CN oder COOH bedeutet, eignen sich ferner als Zwischenprodukte zur Herstellung anderer Substanzen, die sich als Bestandteile flüssigkristalliner Dielektrika verwenden lassen.

Die Verbindungen der Formel I sind in reinem Zustand farblos und bilden flüssigkristalline Mesophasen in einem für die elektrooptische Verwendung günstig gelegenen Temperaturbereich. Chemisch sind sie sehr stabil.

Gegenstand der Erfindung sind somit die Verbindungen der Formel I sowie ein Verfahren zu ihrer Herstellung dadurch gekennzeichnet, daß man eine der Formel I entsprechende Verbindung, die jedoch an Stelle von Wasserstoffatomen (eine) reduzierbare Gruppe(n) und/oder zusätzliche C-C-Bindungen enthalten, mit einem Reduktionsmittel behandelt und daß man gegebenenfalls in einer erhaltenen Verbindung der Formel I den Rest $R^2$ in einen anderen Rest $R^2$ umwandelt.

Weiterhin ist Gegenstand der Erfindung die Verwendung der Verbindungen der Formel I als Komponenten flüssigkristalliner Dielektrika. Gegenstand der Erfindung sind weiterhin flüssigkristalline Dielektrika mit einem Gehalt an mindestens einer Verbindung der Formel I sowie elektrooptische Anzeigeelemente, die derartige Dielektrika enthalten.

Vor- und nachstehend haben $R^1$, $R^2$, $R^3$, Cy und Ph die angegebene Bedeutung, sofern nicht ausdrücklich etwas anderes vermerkt ist.

Die Verbindungen der Formel I umfassen beispielsweise die bevorzugten Verbindungen der Formel I', die der Formel I entspricht, worin jedoch der Rest $R^2$ CN, —$COOR^3$, —O—CO—$R^3$, oder OH bedeutet, insbesondere die bevorzugten Nitrile der Formel Ia, und die bevorzugten Ester der Formeln Ib und Ic:

$$R^1—Cy—Cy—Ph—CN \qquad (Ia)$$

$$R^1—Cy—Cy—Ph—COOR^3 \quad (Ib)$$
$$R^1—Cy—Cy—Ph—O—CO—R^3 \quad (Ic)$$

Weiterhin bevorzugt sind die vor allem als Zwischenprodukte wichtigen Hydroterphenylderivate der Formel Ie :

$$R^1—Cy—Cy—Ph—OH \quad (Ie)$$

In den Verbindungen der Formel I sowie Ia bis Ie sind diejenigen Stereoisomeren bevorzugt, worin die Substituenten an den beiden 1,4-Cyclohexylenresten jeweils in trans-Stellung zueinander stehen.

In den Verbindungen der Formel I können die Alkyl- bzw. O-Alkylreste geradkettig oder verzweigt sein.

Vorzugsweise sind sie geradkettig, haben 2, 3, 4, 5 oder 6 C-Atome und bedeuten demnach bevorzugt Ethyl, Propyl, Butyl, Pentyl, Hexyl, Ethoxy, Propoxy, Butoxy, Pentoxy oder Hexoxy, ferner Methyl, Heptyl, Octyl, Nonyl, Decyl, Methoxy, Heptoxy, Octoxy, Nonoxy oder Decoxy.

Verbindungen der Formeln I sowie Ia bis Ie mit verzweigten Flügelgruppen $R^1$ bzw. $R^2$ können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Verzweigte Gruppen dieser Art enthalten in der Regel nicht mehr als eine Kettenverzweigung. Bevorzugte verzweigte Reste $R^1$ und $R^2$ sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Heptyl (= 1-Methylhexyl), 2-Octyl (= 1-Methylheptyl), 2-Ethyl-pentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy.

Die Verbindungen der Formel I werden nach an sich bekannten Methoden hergestellt, wie sie in der Literatur (z. B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Die Verbindungen der Formel I werden bevorzugt hergestellt, indem man eine Verbindung der Formel II

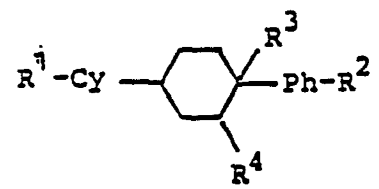

(II)

worin entweder $R^3$ OH und $R^4$ H oder $R^3$ und $R^4$ zusammen eine C-C-Bindung bedeuten, reduziert.

Die Carbinole der Formel II ($R^3$ = OH, $R^4$ = H) sind beispielsweise erhältlich durch Hydrierung von Cyclohexyl-phenolen der Formel $R^1$—Cy—Ph—OH zu Cyclohexylcyclohexanolen der Formel $R^1$—Cy—Cy—OH, Oxydation zu den entsprechenden 4-$R^1$-Cy-cyclohexanonen, Umsetzung mit einer Grignard-Verbindung der Formel BrMg-Ph-$R^2$ und Hydrolyse. Durch Wasserabspaltung, z. B. mit einer starken Säure wie Schwefelsäure oder p-Toluolsulfonsäure in einem inerten Lösungsmittel wie Benzol oder Toluol, können daraus die Cyclohexene der Formel II ($R^3$ und $R^4$ zusammen = C-C-Bindung) hergestellt werden.

Die Reduktion der Ausgangsstoffe der Formel II zu den Verbindungen der Formel I erfolgt zweckmäßig durch katalytische Hydrierung bei Temperaturen zwischen etwa 0° und etwa 200° sowie Drucken zwischen etwa 1 und etwa 200 bar in einem inerten Lösungsmittel, z. B. einem Alkohol wie Methanol, Ethanol oder Isopropanol, einem Ether wie Tetrahydrofuran (THF) oder Dioxan, einem Ester wie Ethylacetat, einer Carbonsäure wie Essigsäure oder einem Kohlenwasserstoff wie Cyclohexan.

Als Katalysatoren eignen sich zweckmäßig Edelmetalle wie Pt oder Pd, die in Form von Oxiden (z. B. $PtO_2$, PdO), auf einem Träger (z. B. Pd auf Kohle, Calciumcarbonat oder Strontiumcarbonat) oder in feinverteilter Form (z. B. Pt-Mohr) eingesetzt werden können.

Nach der Reduktionsstufe kann es zweckmäßig sein, das erhaltene Stereoisomerengemisch in das stabile trans-Isomere (bezogen auf den durch die Hydrierung erhaltenen Cyclohexanring) umzuwandeln, z. B. durch Behandeln mit einer Base wie K-tert.-Butylat in einem inerten Lösungsmittel wie Dimethylformamid (DMF), N-Methylpyrrolidon oder Dimethylsulfoxid bei Temperaturen zwischen etwa 0 und etwa 150°.

Gewünschtenfalls kann man in einer erhaltenen Verbindung der Formel I den Rest $R^2$ in einen anderen Rest $R^2$ umwandeln.

So führt eine Chlorierung oder Bromierung der Hydroterphenyle der Formel I mit $R^2$ = H zu Chlor-

bzw. Bromverbindungen der Formel I ($R^2$ = Cl oder Br), beispielsweise mit elementarem Chlor oder Brom in einem inerten Lösungsmittel wie Diethylether, $CCl_4$ oder Essigsäure bei Temperaturen zwischen etwa —30 und 100°, wobei Katalysatoren wie Eisenspäne, Jod oder $AlCl_3$ zugegen sein können.

Durch Umsetzung dieser Chlor- oder Bromverbindungen mit $Cu_2(CN)_2$, z. B. in Gegenwart von Pyridin in einem inerten Lösungsmittel wie DMF oder N-Methylpyrrolidon bei Temperaturen zwischen 40 und 200°, sind Nitrile der Formel Ia erhältlich.

Weiterhin können Hydroterphenyle der Formel I mit $R^2$ = H durch Acylierung mit Carbonsäuren der Formel HOOC—$R^5$ (worin $R^5$ Alkyl mit 1-9 C-Atomen bedeutet) oder ihren reaktionsfähigen Derivaten in Ketone der Formel $R^1$—Cy—Cy—Ph—CO—$R^5$ übergeführt werden, zweckmäßig in Gegenwart eines sauren Katalysators sowie eines inerten Lösungsmittels bei Temperaturen zwischen etwa 0° und etwa 120°. Als Derivate der Carbonsäuren eignen sich in erster Linie ihre Anhydride und Halogenide, z. B. die entsprechenden Säurechloride und Säurebromide. Als Katalysatoren eignen sich Säuren wie HF, $H_3PO_4$ oder Polyphosphorsäure oder, vorzugsweise, Lewis-Säuren wie $AlCl_3$, $AlBr_3$, $SnCl_4$, $ZnCl_2$, $FeCl_3$, $SbCl_5$, $BF_3$ oder dessen Etherat, als Lösungsmittel z. B. $CS_2$, Kohlenwasserstoffe wie Hexan, halogenierte Kohlenwasserstoffe wie Dichlormethan, Nitrobenzol oder Tetramethylensulfon.

Die genannten Ketone können zu Carbonsäuren der Formel I ($R^2$ = COOH) oxydiert werden, z. B. mit Hypohalogenit, zweckmäßig in situ erzeugt aus Brom und einer Base wie NaOH in wässerigem Dioxan bei Temperaturen zwischen etwa 0 und etwa 50°.

Die Carbonsäuren der Formel I ($R^2$ = COOH) können in üblicher Weise, z. B. mit $SOCl_2$ oder $PCl_5$ bei Temperaturen zwischen etwa 0 und 100°, in die entsprechenden Säurechloride und diese mit Ammoniak, z. B. in wässerigem Dioxan bei Temperaturen zwischen etwa 0 und 30°, in die entsprechenden Säureamide der Formel $R^1$—Cy—Cy—Ph—$CONH_2$ übergeführt werden.

Dehydratisierung dieser Amide führt zu den Nitrilen der Formel Ia. Als wasserabspaltende Mittel eignen sich beispielsweise anorganische Säurechloride wie $SOCl_2$, $PCl_3$, $POCl_3$, $PCl_5$, $SO_2Cl_2$, $COCl_2$, ferner $P_2O_5$, $P_2S_5$, $AlCl_3$ (z. B. als Doppelverbindung mit NaCl), aromatische Sulfonsäuren und Sulfonsäurehalogenide. Man kann dabei in Gegenwart oder Abwesenheit eines inerten Lösungsmittels bei Temperaturen zwischen etwa 20 und 150° arbeiten ; als Lösungsmittel kommen z. B. aromatische Kohlenwasserstoffe wie Benzol, Toluol oder Xylol oder Amide wie DMF in Betracht.

Verbindungen der Formel I mit $R^2$ = Alkyl sind z. B. erhältlich durch Reduktion der genannten Ketone der Formel $R^1$—Cy—Cy—Ph—CO—$R^5$ nach den Methoden von Clemmensen (mit Zink, amalgamierten Zink oder Zinn und Salzsäure, zweckmäßig in wässerig-alkoholischer Lösung oder in heterogener Phase mit Wasser/Benzol oder Wasser/Toluol bei Temperaturen zwischen etwa 80 und 120°) oder Wolff-Kishner (mit Hydrazin, zweckmäßig in Gegenwart von Alkali wie KOH oder NaOH in einem hochsiedenden Lösungsmittel wie Diethylenglykol oder Triethylenglykol bei Temperaturen zwischen etwa 100 und 200°). Die Ketone können auch katalytisch unter den oben angegebener Bedingungen zu den Kohlenwasserstoffen der Formel I mit $R^2$ = Alkyl hydriert werden, bevorzugt an einem Pt- oder Pd-Katalysator bei Temperaturen zwischen 20 und 80° und Normaldruck in einem der genannten Lösungsmittel, z. B. THF.

Umsetzung der genannten Ketone mit Hydroxylamin, z. B. in wässerigem Ethanol in Gegenwart einer Base wie KOH oder Pyridin bei Temperaturen zwischen etwa 20 und 100°, liefert die entsprechenden Oxime, die unter den Bedingungen einer Beckmann-Umlagerung, z. B. mit $PCl_5$ oder Ameisensäure bei Temperaturen zwischen etwa 20 und 100°, in die entsprechenden Amide der Formel $R^1$—Cy—Cy—Ph—NH—CO—$R^5$ umgewandelt werden können. Hydrolyse, z. B. mit wässerigen Mineralsäuren wie Schwefelsäure oder Salzsäure bei Temperaturen zwischen etwa 20 und 150°, führt zu den entsprechenden Aminen der Formel $R^1$—Cy—Cy—Ph—$NH_2$. Diese können auch durch Nitrierung der Kohlenwasserstoffe der Formel I mit $R^2$ = H und anschließende Reduktion der erhaltenen Nitroverbindungen der Formel $R^1$—Cy—Cy—Ph—$NO_2$ hergestellt werden, beispielsweise durch Hydrierung unter den oben angegebenen Bedingungen, oder auch auf chemischem Wege, z. B. mit nascierendem Wasserstoff, der mit den Systemen Fe/HCl, Zn/NaOH, Zn/$CH_3COOH$ oder Sn/HCl erzeugt werden kann, mit $SnCl_2$/HCl, mit $H_2S$ oder Sulfiden oder mit $Na_2S_2O_4$.

Phenole der Formel Ie sind z. B. durch Diazotierung der Amine und nachfolgende Verkochung erhältlich. Man kann wie üblich mit einem Salz oder einem Ester der salpetrigen Säure (wie $NaNO_2$ oder Butylnitrit) in saurem wässerigem Medium diazotieren und die erhaltene Diazoniumsalzlösung anschließend durch Hydrolyse bei Temperaturen zwischen etwa 50 und 150° zersetzen.

Alkoxyverbindungen der Formel I ($R^2$ = O-Alkyl) sind durch Alkylierung der Phenole der Formel Ie erhältlich, wobei das Phenol zweckmäßig zunächst in ein Phenolat, z. B. durch Behandeln mit NaOH, KOH, $Na_2CO_3$ oder $K_2CO_3$ in das entsprechende Alkalimetallphenolat übergeführt wird. Dieses kann dann mit dem entsprechenden Alkylhalogenid, -sulfonat oder Dialkylsulfat umgesetzt werden, zweckmäßig in einem inerten Lösungsmittel wie Aceton, DMF oder Dimethylsulfoxid oder auch einem Überschuß an wässeriger oder wässerig-alkoholischer NaOH oder KOH bei Temperaturen zwischen etwa 20 und 100°.

Ester der Formeln Ib (worin $R^3$ von H verschieden ist) und Ic können auch durch Veresterung von Carbonsäuren der Formel I ($R^2$ = COOH) (oder ihren reaktionsfähigen Derivaten) mit Alkoholen bzw. Phenolen der Formel HO—$R^3$ (oder ihren reaktionsfähigen Derivaten) oder von Phenolen der Formel Ie (oder ihren reaktionsfähigen Derivaten) mit Carbonsäuren der Formel HOOC—$R^3$ (oder ihren reaktionsfähigen Derivaten) erhalten werden.

Als reaktionsfähige Derivate der genannten Carbonsäuren eignen sich insbesondere die Säurehalogenide, vor allem die Chloride und Bromide, ferner die Anhydride, z. B. auch gemischte Anhydride der Formeln $R^1$—Cy—Cy—Ph—CO—O—$COCH_3$ bzw. $R^3$—CO—O—$COCH_3$, Azide, Ester, insbesondere Alkylester mit 1-4 C-Atomen in der Alkylgruppe.

Als reaktionsfähige Derivate der genannten Alkohole bzw. Phenole kommen insbesondere die entsprechenden Metall-alkoholate bzw. -phenolate der Formeln MO—$R^3$ bzw. $R^1$—Cy—Cy—Ph—OM in Betracht, in denen M ein Äquivalent eines Metalls, vorzugsweise eines Alkalimetalls wie Na oder K, bedeutet.

Die Veresterung wird vorteilhaft in Gegenwart eines inerten Lösungsmittels durchgeführt. Gut geeignet sind insbesondere Ether wie Diethylether, Di-n-butylether, THF, Dioxan oder Anisol, Ketone wie Aceton, Butanon oder Cyclohexanon, Amide wie DMF oder Phosphorsäure-hexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuß einer organischen Base, zum Beispiel Pyridin, Chinolin oder Triethylamin als Lösungsmittel für die Veresterung angewandt werden. Die Veresterung kann auch in Abwesenheit eines Lösungsmittels, z. B. durch einfaches Erhitzen der Komponenten in Gegenwart von Natriumacetat, durchgeführt werden. Die Reaktionstemperatur liegt gewöhnlich zwischen —50° und + 250°, vorzugsweise zwischen —20° und + 80°. Bei diesen Temperaturen sind die Veresterungsreaktionen in der Regel nach 15 Minuten bis 48 Stunden beendet.

Im einzelnen hängen die Reaktionsbedingungen für die veresterung weitgehend von der Natur der verwendeten Ausgangsstoffe ab. So wird eine freie Carbonsäure mit einem freien Alkohol oder Phenol in der Regel in Gegenwart einer starken Säure, beispielsweise einer Mineralsäure wie Salzsäure oder Schwefelsäure, umgesetzt. Eine bevorzugte Reaktionsweise ist die Umsetzung eines Säureanhydrids oder insbesondere eines Säurechlorids mit einem Alkohol, vorzugsweise in einem basischen Milieu, wobei als Basen insbesondere Alkalimetallhydroxide wie Natrium- oder Kaliumhydroxid, Alkalimetallcarbonate bzw. -hydrogencarbonate wie Natriumcarbonat, Natriumhydrogencarbonat, Kaliumcarbonat oder Kaliumhydrogencarbonat, Alkalimetallacetate wie Natrium- oder Kaliumacetat, Erdalkalimetallhydroxide wie Calciumhydroxid oder organische Basen wie Triethylamin, Pyridin, Lutidin, Kollidin oder Chinolin von Bedeutung sind. Eine weitere bevorzugte Ausführungsform der Veresterung besteht darin, daß man den Alkohol bzw. das Phenol zunächst in das Natrium- oder Kaliumalkoholat bzw. -phenolat überführt, z. B. durch Behandlung mit ethanolischer Natron- oder Kalilauge, dieses isoliert und zusammen mit Natriumhydrogencarbonat oder Kaliumcarbonat unter Rühren in Aceton oder Diethylether suspendiert und diese Suspension mit einer Lösung des Säurechlorids oder Anhydrids in Diethylether, Aceton oder DMF versetzt, zweckmäßig bei Temperaturen zwischen etwa —25 und + 20°.

Die erfindungsgemäßen Dielektrika bestehen aus 2 bis 15, vorzugsweise 3 bis 12 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexan-carbonsäurephenyl- oder -cyclohexyl-ester, Phenylcyclohexane, Cyclohexylbiphenyle, Cyclohexylcyclohexane, Cyclohexylnaphthaline, 1,4-Bis-cyclohexylbenzole, 4,4'-Biscyclohexylbiphenyle, Phenyl- oder Cyclohexyl-pyrimidine, Phenyl- oder Cyclohexyldioxane, gegebenenfalls halogenierten Stilbene, Benzylphenylether, Tolane und substituierten Zimtsäuren.

Die wichtigsten als Bestandteile derartiger flüssigkristalliner Dielektrika in Frage kommenden Verbindungen lassen sich durch die Formel III charakterisieren,

$$R^6—A—G—E—R^7 \qquad (III)$$

worin A und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

| | | |
|---|---|---|
| G | —CH=CH— | —N(O)=N— |
| G | —CH=CY— | —CH=N(O)— |
| G | —C≡C— | —$CH_2$—$CH_2$— |
| G | —CO—O— | —$CH_2$—O— |
| G | —CO—S— | —$CH_2$—S— |
| G | —CH=N— | —COO—Ph—COO— |

oder eine C-C-Einfachbindung, Y Halogen, vorzugsweise Chlor, oder —CN, und $R^6$ und $R^7$ Alkyl, Alkoxy, Alkanoyloxy oder Alkoxycarbonyloxy mit bis zu 18, vorzugsweise bis zu 8 Kohlenstoffatomen, oder einer dieser Reste auch CN, NC, $NO_2$, $CF_3$, F, Cl oder Br bedeuten.

Bei den meisten dieser Verbindungen sind $R^6$ und $R^7$ voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich.

Die erfindungsgemäßen Dielektrika enthalten etwa 0,1 bis 60, vorzugsweise 2 bis 25 %, einer oder mehrerer Verbindungen der Formel I.

Die Herstellung der erfindungsgemäßen Dielektrika erfolgt in an sich üblicher Weise. In der Regel werden die Komponenten ineinander gelöst, zweckmäßig bei erhöhter Temperatur.

Durch geeignete Zusätze können die flüssigkristallinen Dielektrika nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecylammonium-4-hexyloxybenzoat, Tetrabutylammonium-tetraphenylboranat oder Komplexsalze von Kronenethern (vergl. z. B. I. Haller et al., Mol. Cryst. Liq. Cryst. Band 24, Seiten 249-258 (1973)) zur Verbesserung der Leitfähigkeit, dichroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden. Derartige Substanzen sind zum Beispiel in den DE-OS 22 09 127, 22 40 864, 23 21 632, 23 38 281, 24 50 088, 26 37 430, 28 53 728 und 29 02 177 beschrieben.

Die folgenden Beispiele sollen die Erfindung erläutern, ohne sie zu begrenzen. In den Beispielen bedeuten F. den Schmelzpunkt und K. den Klärpunkt einer flüssigkristallinen Substanz. Vor- und nachstehend bedeuten Prozentangaben Gewichtsprozent ; alle Temperaturer sind in Grad Celsius angegeben. « Übliche Aufarbeitung » bedeutet : man gibt, falls erforderlich, Wasser und/oder ein organisches Lösungsmittel wie Toluol $CH_2Cl_2$ oder $CHCl_3$ hinzu, trennt ab, dampft die organische Phase ein und reinigt den Rückstand durch Chromatographie und/oder Kristallisation.

## Beispiel 1

Eine Lösung von 28,2 g 1-Phenyl-4-(trans-4-propyl-cyclohexyl)-cyclohexen [erhältlich durch Hydrierung von trans-1-p-Hydroxyphenyl-4-propyl-cyclohexan an $PtO_2$ in Ethylacetat bei 50° zu trans-4-(trans-4-Propylcyclohexyl)-cyclohexanol, Oxydation mit NaOCl zu 4-(trans-4-Propylcyclohexyl)-cyclohexanon, Reaktion mit $C_6H_5MgBr$ in THF und nachfolgende Hydrolyse zu 1-Phenyl-4-(trans-4-propylcyclohexyl)-cyclohexanol und Dehydratisierung mit p-Toluolsulfonsäure in siedendem Toluol] in 350 ml THF wird an 3 g PdO bei 40° und 1 bar bis zum Stillstand hydriert. Man filtriert, dampft ein, löst das erhaltene cis-trans-Gemisch in 140 ml DMF, versetzt mit 14 g K-tert.-Butylat und erhitzt 24 Stunden unter $N_2$ auf 100°.

Nach dem Abkühlen gießt man in Wasser und filtriert das erhaltene trans-1-Phenyl-4-(trans-4-propylcyclohexyl)-cyclohexan ab. F. 74 °C, K. 102 °C.

Analog erhält man durch Hydrierung der entsprechenden Cyclohexene :

trans-1-Phenyl-4-(trans-4-methylcyclohexyl)-cyclohexan
trans-1-Phenyl-4-(trans-4-ethylcyclohexyl)-cyclohexan
trans-1-Phenyl-4-(trans-4-butylcyclohexyl)-cyclohexan
trans-1-Phenyl-4-(trans-4-pentylcyclohexyl)-cyclohexan
trans-1-Phenyl-4-(trans-4-hexylcyclohexyl)-cyclohexan
trans-1-Phenyl-4-(trans-4-heptylcyclohexyl)-cyclohexan
trans-1-Phenyl-4-(trans-4-octylcyclohexyl)-cyclohexan
trans-1-Phenyl-4-(trans-4-nonylcyclohexyl)-cyclohexan
trans-1-Phenyl-4-(trans-4-decylcyclohexyl)-cyclohexan
trans-1-p-Tolyl-4-(trans-4-ethylcyclohexyl)-cyclohexan
trans-1-p-Tolyl-4-(trans-4-propylcyclohexyl)-cyclohexan
trans-1-p-Tolyl-4-(trans-4-butylcyclohexyl)-cyclohexan
trans-1-p-Tolyl-4-(trans-4-pentylcyclohexyl)-cyclohexan
trans-1-p-Tolyl-4-(trans-4-hexylcyclohexyl)-cyclohexan
trans-1-p-Ethylphenyl-4-(trans-4-ethylcyclohexyl)-cyclohexan
trans-1-p-Ethylphenyl-4-(trans-4-propylcyclohexyl)-cyclohexan, F. 33 °C, K. 162 °C, $\nu_{20}$ 16 mm2/s
trans-1-p-Ethylphenyl-4-(trans-4-butylcyclohexyl)-cyclohexan
trans-1-p-Ethylphenyl-4-(trans-4-pentylcyclohexyl)-cyclohexan
trans-1-p-Ethylphenyl-4-(trans-4-hexylcyclohexyl)-cyclohexan
trans-1-p-Propylphenyl-4-(trans-4-ethylcyclohexyl)-cyclohexan
trans-1-p-Propylphenyl-4-(trans-4-propylcyclohexyl)-cyclohexan
trans-1-p-Propylphenyl-4-(trans-4-butylcyclohexyl)-cyclohexan
trans-1-p-Propylphenyl-4-(trans-4-pentylcyclohexyl)-cyclohexan
trans-1-p-Propylphenyl-4-(trans-4-hexylcyclohexyl)-cyclohexan
trans-1-p-Butylphenyl-4-(trans-4-ethylcyclohexyl)-cyclohexan
trans-1-p-Butylphenyl-4-(trans-4-propylcyclohexyl)-cyclohexan
trans-1-p-Butylphenyl-4-(trans-4-butylcyclohexyl)-cyclohexan
trans-1-p-Butylphenyl-4-(trans-4-pentylcyclohexyl)-cyclohexan
trans-1-p-Butylphenyl-4-(trans-4-hexylcyclohexyl)-cyclohexan

trans-1-p-Pentylphenyl-4-(trans-4-ethylcyclohexyl)-cyclohexan
trans-1-p-Pentylphenyl-4-(trans-4-propylcyclohexyl)-cyclohexan
trans-1-p-Pentylphenyl-4-(trans-4-butylcyclohexyl)-cyclohexan
trans-1-p-Pentylphenyl-4-(trans-4-pentylcyclohexyl)-cyclohexan
trans-1-p-Pentylphenyl-4-(trans-4-hexylcyclohexyl)-cyclohexan
trans-1-p-Hexylphenyl-4-(trans-4-ethylcyclohexyl)-cyclohexan
trans-1-p-Hexylphenyl-4-(trans-4-propylcyclohexyl)-cyclohexan
trans-1-p-Hexylphenyl-4-(trans-4-butylcyclohexyl)-cyclohexan
trans-1-p-Hexylphenyl-4-(trans-4-pentylcyclohexyl)-cyclohexan
trans-1-p-Hexylphenyl-4-(trans-4-hexylcyclohexyl)-cyclohexan
trans-1-p-Methoxyphenyl-4-(trans-4-ethylcyclohexyl)-cyclohexan
trans-1-p-Methoxyphenyl-4-(trans-4-propylcyclohexyl)-cyclohexan
trans-1-p-Methoxyphenyl-4-(trans-4-butylcyclohexyl)-cyclohexan
trans-1-p-Methoxyphenyl-4-(trans-4-pentylcyclohexyl)-cyclohexan
trans-1-p-Methoxyphenyl-4-(trans-4-hexylcyclohexyl)-cyclohexan
trans-1-p-Ethoxyphenyl-4-(trans-4-ethylcyclohexyl)-cyclohexan
trans-1-p-Ethoxyphenyl-4-(trans-4-propylcyclohexyl)-cyclohexan
trans-1-p-Ethoxyphenyl-4-(trans-4-butylcyclohexyl)-cyclohexan
trans-1-p-Ethoxyphenyl-4-(trans-4-pentylcyclohexyl)-cyclohexan
trans-1-p-Ethoxyphenyl-4-(trans-4-hexylcyclohexyl)-cyclohexan
trans-1-p-Propoxyphenyl-4-(trans-4-ethylcyclohexyl)-cyclohexan
trans-1-p-Propoxyphenyl-4-(trans-4-propylcyclohexyl)-cyclohexan
trans-1-p-Propoxyphenyl-4-(trans-4-butylcyclohexyl)-cyclohexan
trans-1-p-Propoxyphenyl-4-(trans-4-pentylcyclohexyl)-cyclohexan
trans-1-p-Propoxyphenyl-4-(trans-4-hexylcyclohexyl)-cyclohexan
trans-1-p-Butoxyphenyl-4-(trans-4-ethylcyclohexyl)-cyclohexan
trans-1-p-Butoxyphenyl-4-(trans-4-propylcyclohexyl)-cyclohexan
trans-1-p-Butoxyphenyl-4-(trans-4-butylcyclohexyl)-cyclohexan
trans-1-p-Butoxyphenyl-4-(trans-4-pentylcyclohexyl)-cyclohexan
trans-1-p-Butoxyphenyl-4-(trans-4-hexylcyclohexyl)-cyclohexan
trans-1-p-Pentoxyphenyl-4-(trans-4-ethylcyclohexyl)-cyclohexan
trans-1-p-Pentoxyphenyl-4-(trans-4-propylcyclohexyl)-cyclohexan
trans-1-p-Pentoxyphenyl-4-(trans-4-butylcyclohexyl)-cyclohexan
trans-1-p-Pentoxyphenyl-4-(trans-4-pentylcyclohexyl)-cyclohexan
trans-1-p-Pentoxyphenyl-4-(trans-4-hexylcyclohexyl)-cyclohexan
trans-1-p-Hexoxyphenyl-4-(trans-4-ethylcyclohexyl)-cyclohexan
trans-1-p-Hexoxyphenyl-4-(trans-4-propylcyclohexyl)-cyclohexan
trans-1-p-Hexoxyphenyl-4-(trans-4-butylcyclohexyl)-cyclohexan
trans-1-p-Hexoxyphenyl-4-(trans-4-pentylcyclohexyl)-cyclohexan
trans-1-p-Hexoxyphenyl-4-(trans-4-hexylcyclohexyl)-cyclohexan.

## Beispiel 2

a) Ein Gemisch aus 28,4 g trans-1-Phenyl-4-(trans-4-propylcyclohexyl)-cyclohexan, 13,4 g $AlCl_3$ und 120 ml $CH_2Cl_2$ wird bei 20° 24 Std. gerührt und dann in 2%ige Salzsäure gegossen. Nach üblicher Aufarbeitung erhält man p-(trans-4-(trans-4-Propylcyclohexyl)-cyclohexyl)-acetophenon. F. 81°, K. 115°.

b) Eine Lösung von 32,6 g p-(trans-4-(trans-4-Propylcyclohexyl)-cyclohexyl)-acetophenon in 200 ml Ethanol wird mit einer Lösung von 7 g Hydroxylammoniumchlorid in 115 ml Wasser versetzt. Nach Zugabe einer Lösung von 5,6 g KOH in 10 ml Wasser wird 2 Std. gekocht, abgekühlt, mit Wasser verdünnt und das ausgefallene p-(trans-4-(trans-4-Propylcyclohexyl)-cyclohexyl)-acetophenon-oxim abfiltriert, mit Wasser gewaschen und getrocknet.

c) Ein Gemisch aus 34,1 g p-(trans-4-(trans-4-Propylcyclohexyl)-cyclohexyl)-acetophenon-oxim und 170 ml Ameisensäure wird 12 Std. gekocht und eingedampft. Der rohe, aus trans-1-p-Acetamidophenyl-4-(trans-4-propylcyclohexyl)-cyclohexan bestehende Rückstand wird mit 340 ml 20%iger $H_2SO_4$ 3 Std. gekocht, um die Acetylgruppe abzuspalten. Nach dem Abkühlen wird bei 0° eine Lösung von 7 g $NaNO_2$ in 20 ml Wasser unter Rühren zugetropft, 1 Std. nachgerührt und das so erhaltene Gemisch portionsweise unter Rühren in 300 ml auf 130° erwärmte 60 %ige $H_2SO_4$ eingetragen. Man kühlt ab, filtriert das erhaltene trans-1-p-Hydroxyphenyl-4-(trans-4-propylcyclohexyl)-cyclohexan ab und kristallisiert aus Toluol um.

Analog erhält man über

p-(trans-4-(trans-4-Ethylcyclohexyl)-cyclohexyl)-acetophenon
p-(trans-4-(trans-4-Butylcyclohexyl)-cyclohexyl)-acetophenon
p-(trans-4-(trans-4-Pentylcyclohexyl)-cyclohexyl)-acetophenon

p-(trans-4-(trans-4-Hexylcyclohexyl)-cyclohexyl)-acetophenon

sowie die entsprechenden Oxime, über

trans-1-p-Acetamidophenyl-4-(trans-4-ethylcyclohexyl)-cyclohexan
trans-1-p-Acetamidophenyl-4-(trans-4-butylcyclohexyl)-cyclohexan
trans-1-p-Acetamidophenyl-4-(trans-4-pentylcyclohexyl)-cyclohexan
trans-1-p-Acetamidophenyl-4-(trans-4-hexylcyclohexyl)-cyclohexan

und die entsprechenden 1-p-Aminophenylverbindungen

trans-1-p-Hydroxyphenyl-4-(trans-4-ethylcyclohexyl)-cyclohexan
trans-1-p-Hydroxyphenyl-4-(trans-4-butylcyclohexyl)-cyclohexan
trans-1-p-Hydroxyphenyl-4-(trans-4-pentylcyclohexyl)-cyclohexan
trans-1-p-Hydroxyphenyl-4-(trans-4-hexylcyclohexyl)-cyclohexan.

## Beispiel 3

Eine Lösung von 32,6 g p-(trans-4-(trans-4-Propylcyclohexyl-cyclohexyl)-acetophenon in 320 ml THF wird an 3 g 5 %igem Pd—C bei 40° und Normaldruck bis zum Stillstand der $H_2$-Aufnahme hydriert. Man filtriert, dampft ein und erhält trans-1-p-Ethylphenyl-4-(trans-4-propylcyclohexyl)-cyclohexan.

## Beispiel 4

Ein Gemisch aus 32,6 g p-(trans-4-(trans-4-Propylcyclohexyl)-cyclohexyl)-acetophenon, 15 g KOH, 25 ml 85 %igem Hydrazin und 250 ml Diethylenglykol wird 1 Std. auf 100° erwärmt. Man steigert die Temperatur langsam bis zur Zersetzung des gebildeten Hydrazons, kocht noch 4 Std., kühlt ab, arbeitet wie üblich auf und erhält trans-1-p-Ethylphenyl-4-(trans-4-propylcyclohexyl)-cyclohexan.

## Beispiel 5

Ein Gemisch aus 36,3 g trans-1-p-Bromphenyl-4-(trans-4-propylcyclohexyl)-cyclohexan, 10 g $Cu_2(CN)_2$, 120 ml Pyridin und 60 ml N-Methylpyrrolidon wird 2 Std. auf 160° erhitzt. Man kühlt ab, gibt eine Lösung von 120 g $FeCl_3 \cdot 6H_2O$ in 600 ml 20 %iger Salzsäure hinzu, erwärmt 1,5 Std. unter Rühren auf 70°, arbeitet wie üblich auf und erhält trans-1-p-Cyanphenyl-4-(trans-4-propylcyclohexyl)-cyclohexan, F. 55°, K. 184°.

Analog erhält man aus den entsprechenden Bromverbindungen :

trans-1-p-Cyanphenyl-4-(trans-4-methylcyclohexyl)-cyclohexan
trans-1-p-Cyanphenyl-4-(trans-4-ethylcyclohexyl)-cyclohexan, F. 76°, K. 195°
trans-1-p-Cyanphenyl-4-(trans-4-butylcyclohexyl)-cyclohexan
trans-1-p-Cyanphenyl-4-(trans-4-pentylcyclohexyl)-cyclohexan
trans-1-p-Cyanphenyl-4-(trans-4-hexylcyclohexyl)-cyclohexan
trans-1-p-Cyanphenyl-4-(trans-4-heptylcyclohexyl)-cyclohexan
trans-1-p-Cyanphenyl-4-(trans-4-octylcyclohexyl)-cyclohexan
trans-1-p-Cyanphenyl-4-(trans-4-nonylcyclohexyl)-cyclohexan
trans-1-p-Cyanphenyl-4-(trans-4-decylcyclohexyl)-cyclohexan.

## Beispiel 6

Man tropft eine Lösung von 32,6 g p-(trans-4-(trans-4-Propylcyclohexyl)-cyclohexyl)-acetophenon in 100 ml Dioxan bei 20° unter Rühren zu einer Lösung von 40 g NaOH und 48 g Brom in 230 ml Wasser, rührt noch 1 Std., versetzt mit einer Lösung von 14 g $NaHSO_3$ in 140 ml Wasser und gibt Salzsäure bis pH 5 hinzu. Übliche Aufarbeitung ($CH_2Cl_2$) liefert p-(trans-4-(trans-4-Propylcyclohexyl)-cyclohexyl)-benzoesäure. Das Rohprodukt wird mit 60 ml $SOCl_2$ 2,5 Std. gekocht. Nach dem Eindampfen löst man das erhaltene rohe Säurechlorid in 430 ml Dioxan, versetzt mit 200 ml 25 %igem wässerigem Ammoniak, gießt auf Eis, filtriert und erhält p-(trans-4-(trans-4-Propylcyclohexyl)-cyclohexyl)-benzamid. Eine Lösung von 32,7 g dieses Amids in 500 ml DMF wird bei 50° tropfenweise unter Rühren mit 65 g $POCl_3$ versetzt. Nach weiterem einstündigem Rühren gießt man auf Eis, arbeitet wie üblich auf ($CH_2Cl_2$) und erhält trans-1-p-Cyanphenyl-4-(trans-4-propyl-cyclohexyl)-cyclohexan, F. 55°, K. 184°.

Analog erhält man über

p-(trans-4-(trans-4-Ethylcyclohexyl)-cyclohexyl)-benzoesäure
p-(trans-4-(trans-4-Butylcyclohexyl)-cyclohexyl)-benzoesäure
p-(trans-4-(trans-4-Pentylcyclohexyl)-cyclohexyl)-benzoesäure

p-(trans-4-(trans-4-Hexylcyclohexyl)-cyclohexyl)-benzoesäure

die entsprechenden Benzamide und Benzonitrile.

Beispiel 7

Ein Gemisch von 30 g trans-1-p-Hydroxyphenyl-4-(trans-4-propylcyclohexyl)-cyclohexan, 6,9 g $K_2CO_3$, 25 g Hexyljodid und 250 ml DMF wird unter Rühren 16 Std. auf 80° erhitzt, dann abgekühlt und wie üblich aufgearbeitet. Man erhält trans-1-p-Hexoxyphenyl-4-(trans-4-propylcyclohexyl)-cyclohexan.

Beispiel 8

Zu einer Lösung von 30 g trans-1-p-Hydroxyphenyl-4-(trans-4-propylcyclohexyl)-cyclohexan und 20 ml Pyridin in 300 ml Toluol gibt man 8 g Acetylchlorid und erwärmt 1 Std. auf 80°. Nach Abkühlen und üblicher Aufarbeitung erhält man trans-1-p-Acetoxyphenyl-4-(trans-4-propylcyclohexyl)-cyclohexan.

Analog erhält man durch Veresterung

trans-1-p-Acetoxyphenyl-4-(trans-4-ethylcyclohexyl)-cyclohexan
trans-1-p-Formyloxyphenyl-4-(trans-4-propylcyclohexyl)-cyclohexan
trans-1-p-Propionyloxyphenyl-4-(trans-4-propylcyclohexyl)-cyclohexan
trans-1-p-Butyryloxyphenyl-4-(trans-4-propylcyclohexyl)-cyclohexan
trans-1-p-Pentanoyloxyphenyl-4-(trans-4-propylcyclohexyl)-cyclohexan
trans-1-p-Hexanoyloxyphenyl-4-(trans-4-propylcyclohexyl)-cyclohexan
trans-1-p-Decanoyloxyphenyl-4-(trans-4-propylcyclohexyl)-cyclohexan
trans-1-p-(trans-4-Propylcyclohexylcarbonyloxy)-phenyl-4-(trans-4-propylcyclohexyl)-cyclohexan
trans-1-p-(trans-4-Butylcyclohexylcarbonyloxy)-phenyl-4-(trans-4-propylcyclohexyl)-cyclohexan
trans-1-p-(trans-4-Pentylcyclohexylcarbonyloxy)-phenyl-4-(trans-4-propylcyclohexyl)-cyclohexan
trans-1-p-(p-Ethylbenzoyloxy)-phenyl-4-(trans-4-propylcyclohexyl)-cyclohexan
trans-1-p-(p-Propylbenzoyloxy)-phenyl-4-(trans-4-propylcyclohexyl)-cyclohexan
trans-1-p-(p-Butylbenzoyloxy)-phenyl-4-(trans-4-propylcyclohexyl)-cyclohexan
trans-1-p-(p-Methoxybenzoyloxy)-phenyl-4-(trans-4-propylcyclohexyl)-cyclohexan
trans-1-p-(p-Ethoxybenzoyloxy)-phenyl-4-(trans-4-propylcyclohexyl)-cyclohexan
trans-1-p-(p-Propoxybenzoyloxy)-phenyl-4-(trans-4-propylcyclohexyl)-cyclohexan
trans-1-p-(p-Cyanbenzoyloxy)-phenyl-4-(trans-4-propylcyclohexyl)-cyclohexan.

Beispiel 9

Man kocht 32,8 g p-(trans-4-(trans-4-Propylcyclohexyl)-cyclohexyl)-benzoesäure 1 Std. mit 24 g $SOCl_2$, dampft ein, löst das erhaltene rohe Säurechlorid in 150 ml Toluol, versetzt mit 8 g Pyridin und 6 g Propanol und kocht 2 Stunden. Nach Abkühlen und üblicher Aufarbeitung ($CH_2Cl_2$) erhält man p-(trans-4-(trans-4-Propylcyclohexyl)-cyclohexyl)-benzoesäure-propylester.

Analog erhält man durch Veresterung :

p-(trans-4-(trans-4-Ethylcyclohexyl)-cyclohexyl)-benzoesäure-methylester
p-(trans-4-(trans-4-Ethylcyclohexyl)-cyclohexyl)-benzoesäure-ethylester
p-(trans-4-(trans-4-Ethylcyclohexyl)-cyclohexyl)-benzoesäure-propylester
p-(trans-4-(trans-4-Ethylcyclohexyl)-cyclohexyl)-benzoesäure-butylester
p-(trans-4-(trans-4-Ethylcyclohexyl)-cyclohexyl)-benzoesäure-pentylester
p-(trans-4-(trans-4-Ethylcyclohexyl)-cyclohexyl)-benzoesäure-hexylester
p-(trans-4-(trans-4-Ethylcyclohexyl)-cyclohexyl)-benzoesäure-(p-cyanphenylester)
p-(trans-4-(trans-4-Propylcyclohexyl)-cyclohexyl)-benzoesäure-methylester
p-(trans-4-(trans-4-Propylcyclohexyl)-cyclohexyl)-benzoesäure-ethylester
p-(trans-4-(trans-4-Propylcyclohexyl)-cyclohexyl)-benzoesäure-butylester
p-(trans-4-(trans-4-Propylcyclohexyl)-cyclohexyl)-benzoesäure-pentylester
p-(trans-4-(trans-4-Propylcyclohexyl)-cyclohexyl)-benzoesäure-hexylester
p-(trans-4-(trans-4-Propylcyclohexyl)-cyclohexyl)-benzoesäure-heptylester
p-(trans-4-(trans-4-Propylcyclohexyl)-cyclohexyl)-benzoesäure-octylester
p-(trans-4-(trans-4-Propylcyclohexyl)-cyclohexyl)-benzoesäure-nonylester
p-(trans-4-(trans-4-Propylcyclohexyl)-cyclohexyl)-benzoesäure-decylester
p-(trans-4-(trans-4-Propylcyclohexyl)-cyclohexyl)-benzoesäure-(trans-4-propylcyclohexylester)
p-(trans-4-(trans-4-Propylcyclohexyl)-cyclohexyl)-benzoesäure-(trans-4-butylcyclohexylester)
p-(trans-4-(trans-4-Propylcyclohexyl)-cyclohexyl)-benzoesäure-(trans-4-pentylcyclohexylester)
p-(trans-4-(trans-4-Propylcyclohexyl)-cyclohexyl)-benzoesäure-(p-tolylester)
p-(trans-4-(trans-4-Propylcyclohexyl)-cyclohexyl)-benzoesäure-(p-propylphenylester)
p-(trans-4-(trans-4-Propylcyclohexyl)-cyclohexyl)-benzoesäure-(p-butylphenylester)

p-(trans-4-(trans-4-Propylcyclohexyl)-cyclohexyl)-benzoesäure-(p-pentylphenylester)
p-(trans-4-(trans-4-Propylcyclohexyl)-cyclohexyl)-benzoesäure-(p-methoxyphenylester)
p-(trans-4-(trans-4-Propylcyclohexyl)-cyclohexyl)-benzoesäure-(p-ethoxyphenylester)
p-(trans-4-(trans-4-Propylcyclohexyl)-cyclohexyl)-benzoesäure-(p-decoxyphenylester)
p-(trans-4-(trans-4-Propylcyclohexyl)-cyclohexyl)-benzoesäure-(p-cyanphenylester)
p-(trans-4-(trans-4-Butylcyclohexyl)-cyclohexyl)-benzoesäure-propylester
p-(trans-4-(trans-4-Butylcyclohexyl)-cyclohexyl)-benzoesäure-butylester
p-(trans-4-(trans-4-Butylcyclohexyl)-cyclohexyl)-benzoesäure-pentylester
p-(trans-4-(trans-4-Butylcyclohexyl)-cyclohexyl)-benzoesäure-(p-cyanphenylester)
p-(trans-4-(trans-4-Pentylcyclohexyl)-cyclohexyl)-benzoesäure-propylester
p-(trans-4-(trans-4-Pentylcyclohexyl)-cyclohexyl)-benzoesäure-butylester
p-(trans-4-(trans-4-Pentylcyclohexyl)-cyclohexyl)-benzoesäure-pentylester
p-(trans-4-(trans-4-Pentylcyclohexyl)-cyclohexyl)-benzoesäure-(p-cyanphenylester)
p-(trans-4-(trans-4-Hexylcyclohexyl)-cyclohexyl)-benzoesäure-propylester
p-(trans-4-(trans-4-Hexylcyclohexyl)-cyclohexyl)-benzoesäure-butylester
p-(trans-4-(trans-4-Hexylcyclohexyl)-cyclohexyl)-benzoesäure-pentylester
p-(trans-4-(trans-4-Hexylcyclohexyl)-cyclohexyl)-benzoesäure-(p-cyanphenylester).

Es folgen Beispiele für erfindungsgemäße Dielektrika mit einem Gehalt an mindestens einer Verbindung der Formel I :

## Beispiel A

Ein Gemisch aus

21 % p-(trans-4-Propylcyclohexyl)-benzonitril
30 % p-(trans-4-Pentylcyclohexyl)-benzonitril
22 % p-(trans-4-Heptylcyclohexyl)-benzonitril
12 % trans-1-p-Cyanphenyl-4-(trans-4-ethylcyclohexyl)-cyclohexan
15 % trans-1-p-Cyanphenyl-4-(trans-4-propylcyclohexyl)-cyclohexan

zeigt F. — 12°, K. 82°.

Es kann z. B. verwendet werden für einen « Twisted Nematic Display » (TND) bei Ansteuerung im « Static Drive » (« Direktansteuerung ») mit Betriebsspannungen von 3-10 Volt.

## Beispiel B

20 % p-(trans-4-Ethylcyclohexyl)-benzonitril
13 % 4-Ethyl-4'-cyanbiphenyl
22 % 4-Butyl-4'-cyanbiphenyl
9 % p-Ethylbenzoesäure-(p-cyanphenylester)
6 % p-Propylbenzoesäure-(p-cyanphenylester)
13 % trans-1-p-Cyanphenyl-4-(trans-4-ethylcyclohexyl)-cyclohexan
17 % trans-1-p-Cyanphenyl-4-(trans-4-propylcyclohexyl)-cyclohexan

zeigt F. — 8°, K. 64°.

Es kann z. B. verwendet werden für einen TND bei niedrigen Betriebsspannungen.

## Beispiel C

Ein Gemisch aus

12 % p-(trans-4-Propylcyclohexyl)-benzonitril
10 % p-(trans-4-Butylcyclohexyl)-benzonitril
19 % p-(trans-4-Pentylcyclohexyl)-benzonitril
10 % p-(trans-4-Heptylcyclohexyl)-benzonitril
12 % 4-Cyan-4'-(trans-4-pentylcyclohexyl)-biphenyl
12 % trans-1-p-Cyanphenyl-4-(trans-4-ethylcyclohexyl)-cyclohexan
15 % trans-1-p-Cyanphenyl-4-(trans-4-propylcyclohexyl)-cyclohexan
10 % trans-1-p-Fluorphenyl-4-(trans-4-propylcyclohexyl)-cyclohexan

zeigt F. — 13°, K. 97°.

Es ist z. B. als nematisches Lösungsmittel für dichroitische Farbstoffe geeignet (« Hostmischung »).

**Patentansprüche** (für die Vertragsstaaten : FR, IT, NL)

1. Hydroterphenyle der Formel I

$$R^1—Cy—Cy—Ph—R^2 \quad (I)$$

worin
$R^1$ Alkyl,
$R^2$ H, OH, CN, Alkyl, —O-Alkyl, —$COOR^3$, oder —O—CO—$R^3$,
$R^3$ H, Alkyl, —Cy-Alkyl, —Ph-Alkyl, —Ph—O-Alkyl, —Ph—CN oder —Ph—F,
Cy 1,4-Cyclohexylen und
Ph 1,4-Phenylen
bedeuten, wobei die Alkylgruppen jeweils 1-10 C-Atome enthalten.

2. Verfahren zur Herstellung von Hydroterphenylen der Formel I nach Anspruch 1, dadurch gekennzeichnet, daß man eine der Formel I entsprechende Verbindung, die jedoch an Stelle von Wasserstoffatomen (eine) reduzierbare Gruppe(n) und/oder zusätzliche C-C-Bindungen enthalten, mit einem Reduktionsmittel behandelt und daß man gegebenenfalls in einer erhaltenen Verbindung der Formel I den Rest $R^2$ in einen anderen Rest $R^2$ umwandelt.

3. Verwendung der Verbindungen der Formel I nach Anspruch 1 als Komponenten flüssigkristalliner Dielektrika für elektrooptische Anzeigeelemente.

4. Flüssigkristallines Dielektrikum für elektrooptische Anzeigeelemente mit mindestens zwei flüssigkristallinen Komponenten, dadurch gekennzeichnet, daß mindestens eine Komponente eine Verbindung der Formel I nach Anspruch 1 ist.

5. Elektrooptisches Anzeigeelement, dadurch gekennzeichnet, daß es ein Dielektrikum nach Anspruch 4 enthält.

**Patentansprüche** (für die Vertragsstaaten : CH, DE, GB, LI)

1. Hydroterphenyle der Formel I

$$R^1—Cy—Cy—Ph—R^2 \quad (I)$$

worin
$R^1$ Alkyl,
$R^2$ OH, CN, —$COOR^3$ oder —O—CO—$R^3$,
$R^3$ H, Alkyl, —Cy-Alkyl, —Ph-Alkyl, —Ph—O-Alkyl, —Ph—CN oder —Ph—F,
Cy 1,4-Cyclohexylen und
Ph 1,4-Phenylen
bedeuten, wobei die Alkylgruppen jeweils 1-10 C-Atome enthalten.

2. Verfahren zur Herstellung von Hydroterphenylen der Formel I nach Anspruch 1, dadurch gekennzeichnet, daß man eine der Formel I entsprechende Verbindung, die jedoch an Stelle von Wasserstoffatomen (eine) reduzierbare Gruppe(n) und/oder zusätzliche C-C-Bindungen enthalten, mit einem Reduktionsmittel behandelt und daß man gegebenenfalls in einer erhaltenen Verbindung der Formel I den Rest $R^2$ in einen anderen Rest $R^2$ umwandelt.

3. Verwendung der Verbindungen der Formel I nach Anspruch 1 als Komponenten flüssigkristalliner Dielektrika für elektrooptische Anzeigeelemente.

4. Flüssigkristallines Dielektrikum für elektrooptische Anzeigeelemente mit mindestens zwei flüssigkristallinen Komponenten, dadurch gekennzeichnet, daß mindestens eine Komponente eine Verbindung der Formel I nach Anspruch 1 ist.

5. Elektrooptisches Anzeigeelement, dadurch gekennzeichnet, daß es ein Dielektrikum nach Anspruch 4 enthält.

**Patentansprüche** (für den Vertragsstaat AT)

1. Verfahren zur Herstellung von Hydroterphenylen der Formel I

$$R^1—Cy—Cy—Ph—R^2 \quad (I)$$

worin
$R^1$ Alkyl,
$R^2$ H, OH, CN, Alkyl, —O-Alkyl, —$COOR^3$ oder —O—CO—$R^3$,
$R^3$ H, Alkyl, —Cy-Alkyl, —Ph-Alkyl, —Ph—O-Alkyl, —Ph—CN oder —Ph—F,
Cy 1,4-Cyclohexylen und
Ph 1,4-Phenylen

bedeuten, wobei die Alkylgruppen jeweils 1-10 C-Atome enthalten, dadurch gekennzeichnet, daß man eine der Formel I entsprechende Verbindung, die jedoch an Stelle von Wasserstoffatomen (eine) reduzierbare Gruppe(n) und/oder zusätzliche C-C-Bindungen enthalten, mit einem Reduktionsmittel behandelt und daß man gegebenenfalls in einer erhaltenen Verbindung der Formel I den Rest $R^2$ in einen anderen Rest $R^2$ umwandelt.

2. Verwendung der Verbindungen der Formel I nach Anspruch 1 als Komponenten flüssigkristalliner Dielektrika für elektrooptische Anzeigeelemente.

3. Flüssigkristallines Dielektrikum für elektrooptische Anzeigeelemente mit mindestens zwei flüssigkristallinen Komponenten, dadurch gekennzeichnet, daß mindestens eine Komponente eine Verbindung der Formel I nach Anspruch 1 ist.

4. Elektrooptisches Anzeigeelement, dadurch gekennzeichnet, daß es ein Dielektrikum nach Anspruch 3 enthält.

**Claims** (for the Contracting State : FR, IT, NL)

1. Hydroterphenyls of the formula I

$$R^1—Cy—Cy—PH—R^2 \quad (I)$$

wherein
$R^1$ is alkyl,
$R^2$ is H, OH, CN, alkyl, —O-alkyl, —$COOR^3$ or —O—CO—$R^3$,
$R^3$ is H, alkyl, —Cy-alkyl, —Ph-alkyl, —Ph—O-alkyl, —Ph—CN or —Ph—F,
Cy is 1,4-cyclohexylene and
Ph is 1,4-phenylene,
and the alkyl groups each contain 1-10 C atoms.

2. Process for the preparation of hydroterphenyls of the formula I according to Claim 1, characterised in that a compound which corresponds to the formula I but contains (a) reduceable group(s) and/or additional C-C bonds instead of hydrogen atoms, is treated with a reducing agent, and in that, where relevant, the radical $R^2$ in a resulting compound of the formula I is converted into another radical $R^2$.

3. Use of the compounds of the formula I according to Claim 1 as components of liquid crystal dielectrics for electrooptical display elements.

4. Liquid crystal dielectric for electrooptical display elements with at least two liquid crystal components, characterised in that at least one component is a compound of the formula I according to Claim 1.

5. Electrooptical display element, characterised in that it contains a dielectric according to Claim 4.

**Claims** (for the Contracting State: CH, DE, GB, LI)

1. Hydroterphenyls of the formula I

$$R^1—Cy—Cy—Ph—R^2 \quad (I)$$

wherein
$R^1$ is alkyl,
$R^2$ is OH, CN, —$COOR^3$ or —O—CO—$R^3$,
$R^3$ is H, alkyl, —Cy-alkyl, —Ph-alkyl, —Ph—O-alkyl, —Ph—CN or —Ph—F,
Cy is 1,4-cyclohexylene and
Ph is 1,4-phenylene,
and the alkyl groups each contain 1-10 C atoms.

2. Process for the preparation of hydroterphenyls of the formula I according to Claim 1, characterised in that a compound which corresponds to the formula I but contains (a) reduceable group(s) and/or additional C-C bonds instead of hydrogen atoms, is treated with a reducing agent, and in that, where relevant, the radical $R^2$ in a resulting compound of the formula I is converted into another radical $R^2$.

3. Use of the compounds of the formula I according to Claim 1 as components of liquid crystal dielectrics for electrooptical display elements.

4. Liquid crystal dielectric for electrooptical display elements with at least two liquid crystal components, characterised in that at least one component is a compound of the formula I according to Claim 1.

5. Electrooptical display element, characterised in that it contains a dielectric according to Claim 4.

**Claims** (for Contracting State AT)

1. Process for the preparation of hydroterphenyls of the formula I

$$R^1\text{—Cy—Cy—Ph—}R^2 \quad (I)$$

wherein
$R^1$ is alkyl,
$R^2$ is H, OH, CN, Alkyl, —O-alkyl, —$COOR^3$ or —O—CO—$R^3$,
$R^3$ is H, alkyl, —Cy-alkyl, —Ph-alkyl, —Ph—O-alkyl, —Ph—CN or —Ph—F,
Cy is 1,4-cyclohexylene and
Ph is 1,4-phenylene,
and the alkyl groups each contain 1-10 C atoms, characterised in that a compound which corresponds to the formula I but contains (a) reduceable group(s) and/or additional C-C bonds instead of hydrogen atoms, is treated with a reducing agent, and in that, where relevant, the radical $R^2$ in a resulting compound of the formula I is converted into another radical $R^2$.

2. Use of the compounds of the formula I according to Claim 1 as components of liquid crystal dielectrics for electrooptical display elements.

3. Liquid crystal dielectric for electrooptical display elements with at least two liquid crystal components, characterised in that at least one component is a compound of the formula I according to Claim 1.

4. Electrooptical display element, characterised in that it contains a dielectric according to Claim 3.

**Revendications** (pour les Etats contractants : FR, IT, NL)

1. Hydroterphényles de formule I

$$R^1\text{—Cy—Cy—Ph—}R^2 \quad (I)$$

dans laquelle
$R^1$ représente un groupe alkyle,
$R^2$ représente H, OH, CN, un groupe alkyle, O-alkyle, —$COOR^3$ ou —O—CO—$R^3$ ;
$R^3$ représente H, un groupe alkyle, —Cy-alkyle, Ph-alkyle, Ph—O-alkyle, —Ph—CN ou —Ph—F ;
Cy représente un groupe 1,4-cyclohexylène ; et
Ph représente un groupe 1,4-phénylène ;
les groupes alkyle contenant chacun 1 à 10 atomes de carbone.

2. Procédé de préparation des hydroterphényles de formule I selon la revendication 1, caractérisé en ce que l'on traite par un agent réducteur un composé répondant à la formule I mais qui porte à la place des atomes d'hydrogène un ou plusieurs groupes réductibles et/ou des liaisons C-C supplémentaires, et le cas échéant, dans un composé de formule I ainsi obtenu, on convertit le substituant $R^2$ en un autre substituant $R^2$.

3. Utilisation des composés de formule I selon la revendication 1 en tant que composants diélectriques à cristaux liquides pour des éléments d'affichage électro-optiques.

4. Diélectrique à cristaux liquides pour éléments d'affichage électro-optiques contenant au moins deux composants à cristaux liquides, caractérisé en ce que l'un au moins des composants est un composé de formule I selon la revendication 1.

5. Elément d'affichage électro-optique, caractérisé en ce qu'il contient un diélectrique selon la revendication 4.

**Revendications** (pour les Etats contractants : CH, DE, GB, LI)

1. Hydroterphényles de formule I

$$R^1\text{—Cy—Cy—Ph—}R^2 \quad (I)$$

dans laquelle
$R^1$ représente un groupe alkyle,
$R^2$ représente OH, CN, —$COOR^3$ ou —O—CO—$R^3$ ;
$R^3$ représente H, un groupe alkyle, —Cy-alkyle, Ph-alkyle, Ph—O-alkyle, —Ph—CN ou —Ph—F ;
Cy représente un groupe 1,4-cyclohexylène ; et
Ph représente un groupe 1,4-phénylène ;
les groupes alkyle contenant chacun 1 à 10 atomes de carbone.

2. Procédé de préparation des hydroterphényles de formule I selon la revendication 1, caractérisé en

ce que l'on traite par un agent réducteur un composé répondant à la formule I mais qui porte à la place des atomes d'hydrogène un ou plusieurs groupes réductibles et/ou des liaisons C-C supplémentaires, et le cas échéant, dans un composé de formule I ainsi obtenu, on convertit le substituant $R^2$ en un autre substituant $R^2$.

3. Utilisation des composés de formule I selon la revendication 1 en tant que composants diélectriques à cristaux liquides pour des éléments d'affichage électro-optiques.

4. Diélectrique à cristaux liquides pour éléments d'affichage électro-optiques contenant au moins deux composants à cristaux liquides, caractérisé en ce que l'un au moins des composants est un composé de formule I selon la revendication 1.

5. Elément d'affichage électro-optique, caractérisé en ce qu'il contient un diélectrique selon la revendication 4.

**Revendications** (pour l'Etat contractant AT)

1. Procédé de préparation des hydroterphényles de formule I

$$R^1—Cy—Cy—Ph—R^2 \qquad (I)$$

dans laquelle

$R^1$ représente un groupe alkyle,

$R^2$ représente H, OH, CN, un groupe alkyle, O-alkyle, $—COOR^3$ ou $—O—CO—R^3$ ;

$R^3$ représente H, un groupe alkyle, —Cy-alkyle, Ph-alkyle, Ph—O-alkyle, —Ph—CN ou —Ph—F ;

Cy représente un groupe 1,4-cyclohexylène ; et

Ph représente un groupe 1,4-phénylène ;

les groupes alkyle contenant chacun 1 à 10 atomes de carbone, caractérisé en ce que l'on traite par un agent réducteur un composé répondant à la formule I mais qui porte à la place des atomes d'hydrogène un ou plusieurs groupes réductibles et/ou des liaisons C-C supplémentaires, et le cas échéant, dans un composé de formule I ainsi obtenu, on convertit le substituant $R^2$ en un autre substituant $R^2$.

2. Utilisation des composés de formule I selon la revendication 1 en tant que composants diélectriques à cristaux liquides pour des éléments d'affichage électro-optiques.

3. Diélectrique à cristaux liquides pour éléments d'affichage électro-optiques contenant au moins deux composants à cristaux liquides, caractérisé en ce que l'un au moins des composants est un composé de formule I selon la revendication 1.

4. Elément d'affichage électro-optique, caractérisé en ce qu'il contient un diélectrique selon la revendication 3.